# EUROPÄISCHE PATENTSCHRIFT

(11) **EP 1 382 267 B1**
(45) Veröffentlichungstag und Bekanntmachung des Hinweises auf die Patenterteilung: **16.01.2008**
(21) Anmeldenummer: 03016036.0
(22) Anmeldetag: 15.07.2003
(51) Int. Cl.: A41D 27/08, D06H 1/00, G07C 9/00

(54) **Kennzeichnungseinrichtung und Verwendung derselben**
Marking device and use of such a device
Dispositif de marquage et usage d'un tel dispositif

(30) Priorität: 16.07.2002 DE 10232197
(43) Veröffentlichungstag der Anmeldung: 21.01.2004
(73) Patentinhaber: Seitz, Peter, D-81675 München (DE)
(72) Erfinder: Seitz, Peter, D-81675 München (DE)
(74) Vertreter: Bohnenberger, Johannes

(56) Entgegenhaltungen:
- DE-A- 3 149 958
- DE-A- 10 029 854
- US-A- 4 857 916
- US-A- 5 375 397
- US-A- 5 454 600
- US-A- 5 479 528
- US-A- 5 902 111
- PATENT ABSTRACTS OF JAPAN Bd. 0134, Nr. 56 (P-945), 16. Oktober 1989 (1989-10-16) & JP 1 178836 A (ANIMA KK), 17. Juli 1989 (1989-07-17)

## Beschreibung

Die Erfindung betrifft eine Kennzeichnungseinrichtung zum individualisierenden Kennzeichnen von von einer Person am Körper zu tragenden Gegenständen z.B. Bekleidungsstücken Schuhen, Schuheinlagen nach dem Oberbegriff des Patentanspruches 1.

Durch die industrielle Massenproduktion wird es immer schwieriger, "seine" (Marken-) Turnschuhe von denen des Sportkameraden zu unterscheiden, der die selbe Marke gewählt hat. Mit Trikots, Hosen, Handschuhen oder dergleichen ist das nicht anders.

Eine Möglichkeit zur Kennzeichnung z. B. eines textilen Kleidungsstücks besteht im Einsticken eines Monogramms. Dies ist aber außerordentlich aufwendig und bedarf auch immer eines näheren Hinsehens. Darüber hinaus ist die Wahrscheinlichkeit sehr groß, dass verschiedene Personen mit an sich verschiedenen Namen das selbe Monogramm haben.

Aus der US-A-5 454 600 ist es bekannt, auf einem Kleidungsstück einen Fingerabdruck abzubilden, um das Kleidungsstück identifizieren zu können. Ein mehr spaßiger Vorschlag, ein Kleidungsstück mit Handabdrücken zu versehen, ist der US-A-5 902 111 zu entnehmen, wobei hier der Handabdruck nur zu designerischen Zwecken verwendet wird.

Aus der DE-A-100 29 854 ist es bekannt, z.B. auf einem Bekleidungsstück eine bildliche Aufzeichnung einer physiologischen Funktion, wie Augenbewegung, EKG, EEG, EMG oder auch Aufnahmen des Körperinneren anzubringen.

Der Erfindung liegt die Aufgabe zu Grunde, eine Kennzeichnungseinrichtung und ein Verfahren zur Herstellung einer solchen anzugeben, das in einfacher Weise eine leichte Wiedererkennbarkeit bei hoher Individualisierungsfunktion gewährleistet.

Diese Aufgabe wird durch eine Kennzeichnungseinrichtung nach Anspruch 1 bzw. ein Verfahren nach Anspruch 9 gelöst.

Überraschenderweise hat es sich gezeigt, dass ein solches, aus einer begrenzten Anzahl von Punkten- oder Flächenelementen bestehendes "Muster" zum einen einen sehr hohen Wiedererkennungswert aufweist, zum anderen fast schon so personentypisch ist wie ein Fingerabdruck. Während ein Fingerabdruck nur durch Spezialisten von dem Fingerabdruck eines anderen zu unterscheiden ist, gelingt die Unterscheidung von Druckverteilungsmustem verschiedener Personen außerordentlich leicht. Vorrichtungen zur Durchführung dieses Messverfahrens sind z. B. in der EP 0264047 B1 und in den dort zitierten weiteren Schriften offenbart.

Besonders einfach wird die Unterscheidung beziehungsweise hoch wird die Individualisierungsfunktion dann, wenn die verschieden hohe Drücke durch Flächenelemente verschiedener Farben oder verschiedener Schraffuren wiedergegeben sind. Im Gegensatz zu einem Fingerabdruck steht dann nämlich eine dritte Dimension, eben die Farbe (beziehungsweise das Muster) zur Verfügung, wobei derartige Farb- oder Strukturmusterverteilungen vom Menschen sehr gut memorisierbar und von anderen Mustern unterscheidbar sind.

Die Abtastung der Druckverteilung umfasst vorzugsweise eine Vielzahl von Abtastungen von verschiedenen Druckverteilungen, die während eines Bewegungsablaufes beim Aufbau und Abbau eines Kontaktes zwischen dem Körperteil und dem im wesentlichen festen Körper auftreten. Sehr gut vorstellbar wird dies beispielsweise, wenn man sich den Bewegungsablauf und die sich während der Bewegung ändernden (zweidimensionalen) Druckverteilungen unter dem Fuß beim Gehen vorstellt. Man kann nun beispielsweise die Mittelwerte der Druckverteilungen an jedem Messpunkt über die Gesamtzeit des Bewegungsablaufes oder aber auch die Spitzenwerte erfassen und in Farbwerte (verschiedene Schraffuren) umsetzen, die dann zu einem "statischen" Bild zusammengesetzt werden, welches die Kennzeichnungseinrichtung schon darstellen kann.

Eine solche Kennzeichnungseinrichtung beziehungsweise ein solches Bild kann dann zum Beispiel direkt auf ein Kleidungsstück aufgedruckt oder aber durch einen handelsüblichen Drucker auf Folie gedruckt und auf das Kleidungsstück "aufgebügelt" werden.

Besonders sinnvoll und vorteilhaft ist die Verwendung der Kennzeichnungseinrichtung zur Kennzeichnung eines Schuhs oder einer Einlegesohle, wobei das Druckverteilungsmuster unter dem Fuß beim Gehen gewonnen wird. Nachdem das Druckverteilungsmuster insbesondere bei dynamischen Prozessen (wie zum Beispiel beim Gehen) sehr personentypisch ist, muss man das Muster nicht speichern, um es beim Neukauf von Schuhen wieder verwenden zu können, man kann vielmehr das Muster jedes Mal zum Beispiel im Schuhgeschäft neu herstellen lassen, was besonders einfach ist. Das sich ergebende Muster wird dem früher gewonnenen Muster wieder erkennbar gleichen. Es kann hierbei das Muster sowohl in verkleinerter Form oder aber die gesamte Einlegesohle maßstabsgerecht überdeckend aufgebracht sein.

Bei der Verwendung der Kennzeichnungseinrichtung zum Kennzeichnen einer Hose wird zum Beispiel die Druckverteilung beim Hinsetzen und wieder Aufstehen (oder während Teilabschnitten des Bewegungsablaufes) oder aber ein statisches Muster der Druckverteilung bestimmt. Dieses Druckverteilungsmuster kann als Kennzeichnungseinrichtung wieder auf das Bekleidungsstück maßstabsgerecht oder in jeder beliebigen Verkleinerung/Vergrößerung aufgedruckt werden. Gleiches gilt für Handschuhe, die dann auf den Handflächen (oder auch auf dem Handrücken) die Druckverteilung beim Ergreifen eines Gegenstandes oder festen Aufdrücken der Handflächen auf einen Gegenstand aufgedruckt tragen können.

Nachfolgend wird die Erfindung anhand von Abbildungen näher erläutert. Hierbei zeigen
Fig. 1 eine Kennzeichnungseinrichtung mit einem ersten "Fußabdruck",
Fig. 2 eine Kennzeichnungseinrichtung mit einem Fußabdruck einer anderen Person,
Fig. 3 den Fußabdruck gemäß Fig. 1 in einer anderen Darstellungsweise,
Fig. 4 eine Kennzeichnungseinrichtung mit einem Sitz-Verteilungsmuster und
Fig. 5 eine Kennzeichnungseinrichtung mit einer Hand-Druckverteilung.

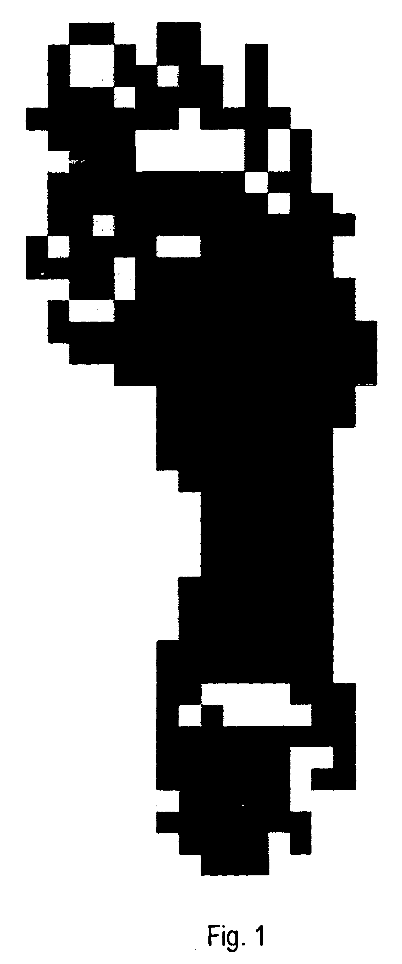

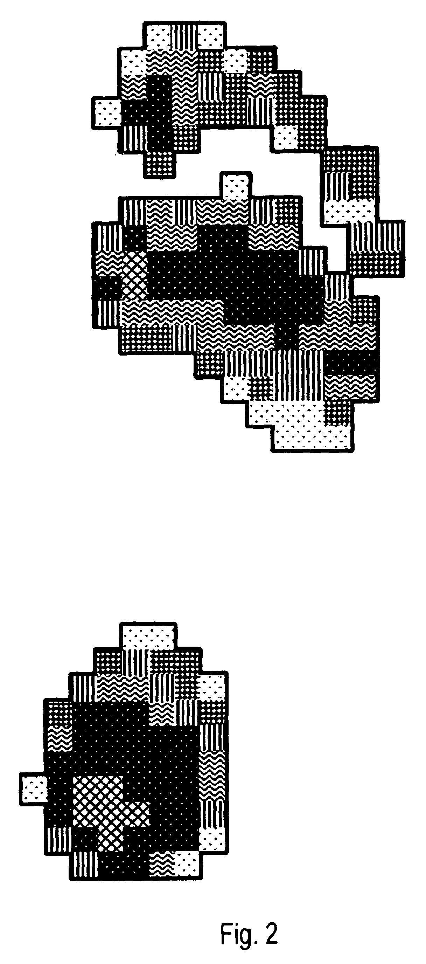

Die in Fig. 1 und in Fig. 2 gezeigten Druckverteilungsmuster stellen Spitzenwerte dar, die beim Abrollen des Fußes auf einer Unterlage während eines Schreitvorganges auftreten. Wie aus den Abbildungen ersichtlich, sind die Druckverteilungsmuster sehr unterschiedlich. Dies liegt nun nicht etwa an den verschiedenen Fußformen. Deren Unterschiede wären sehr viel geringer und wären auch nur schwer erkennbar. Durch das hier verwendete besondere Verfahren zur Herstellung der Abbildung beziehungsweise der Kennzeichnungseinrichtung wird vielmehr der individuelle Bewegungsablauf, also das "Muskelaktivierungsmuster" mit erfasst, so dass nicht nur die Form (wie beim Fingerabdruck), sondern auch die Art der "Benutzung" des Körpers durch die Person wiedergegeben wird.

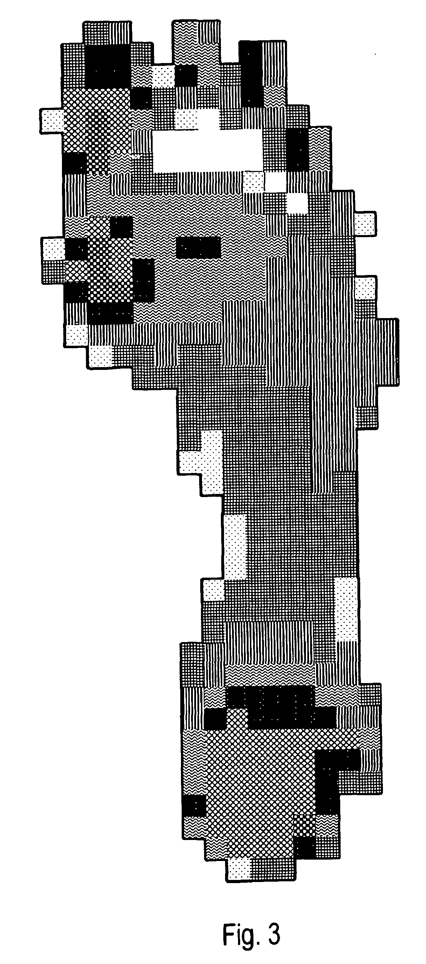

Die in Fig. 3 gezeigte Darstellung unterscheidet sich von der nach Fig. 1 dadurch, dass anstelle von Farbwerten oder - wie in den beiliegenden Abbildungen aus technischen Gründen nicht anders möglich - von Grauwerten verschiedene Schraffuren den gemessenen Druckwerten entsprechen. Der Wiedererkennungswert ist auch hier sehr groß.

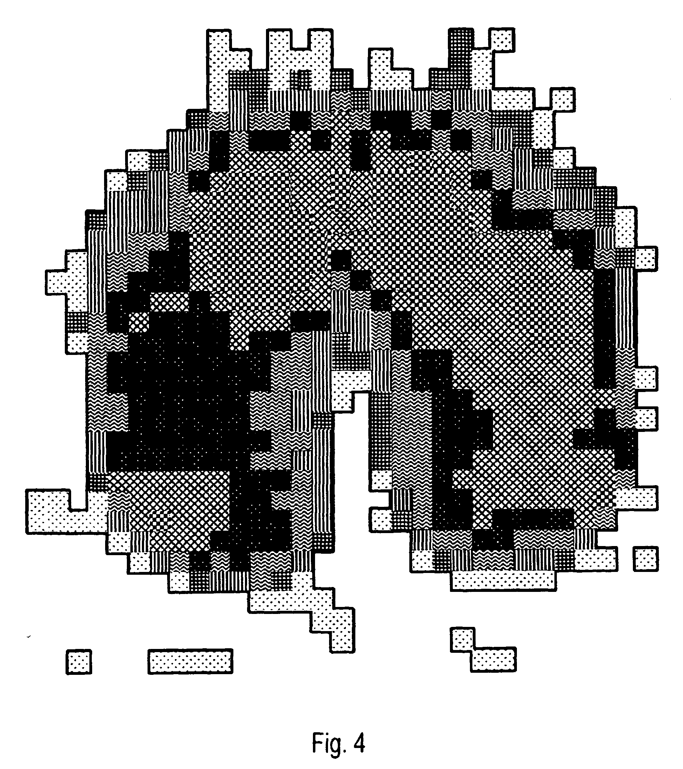

Bei der in Fig. 4 gezeigten Abbildung handelt es sich um ein Abbild der Druckverteilung auf einem (Rollstuhl-)Sitz. Auch hier unterscheiden sich verschiedene Personen erstaunlich stark voneinander, da auch hier das Muskelspiel, die Elastizität/Steifheit der verschiedenen Körperzonen usw. mit in die Abbildung einfließen.

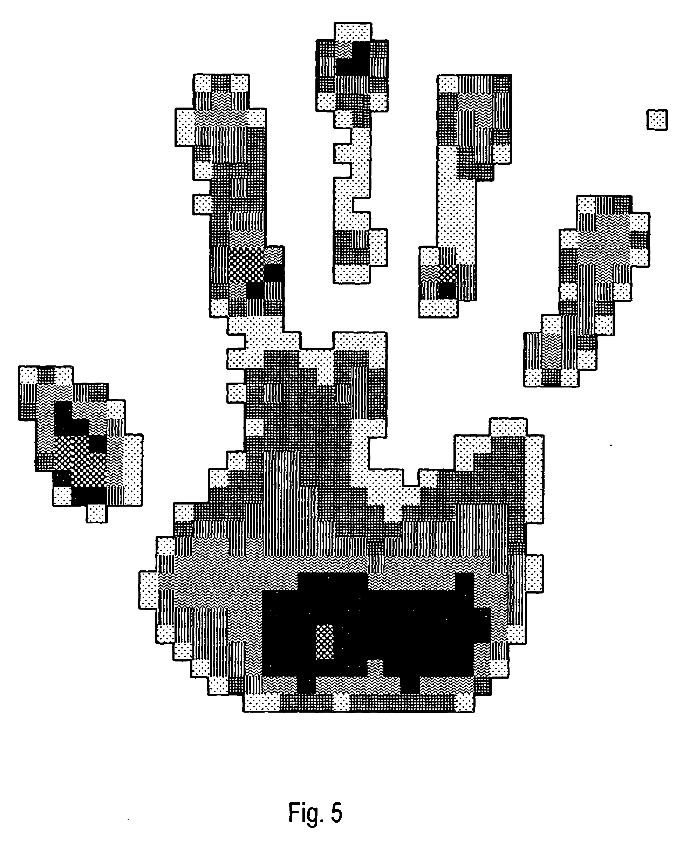

Bei der in Fig. 5 gezeigten Darstellung handelt es sich um die Druckverteilung zwischen einer Handfläche und einer (steifen) Unterlage, die beispielsweise als Kennzeichnungseinrichtung zur Kennzeichnung von Handschuhen Verwendung finden kann. Insbesondere ist die Kennzeichnung eines Handschuhs auf der Handinnenfläche oder aber auch auf der Handaußenfläche in einer im wesentlichen 1:1-Wiedergabe bevorzugt, da so die Wiedererkennung auch unter einer Vielzahl von gleichartig bedruckten Handschuhen sehr gut möglich ist.

Es sei aber an dieser Stelle noch betont, dass ein "Fußabdruck" gemäß einer der Fig. 1 bis 3 nicht nur zur Kennzeichnung von Schuhen oder Einlegesohlen sondern auch zur Kennzeichnung anderer Kleidungsstücke sehr gut verwendbar ist, da der Wiedererkennungswert derartiger Fußabbildungen sehr hoch und die Unterschiede zwischen den in der genannten Art und Weise gewonnenen Abbildungen von verschiedenen Personen sehr groß sind.

## Patentansprüche

1. Kennzeichnungseinrichtung zum individualisierenden Kennzeichnen von von einer Person am Körper zu tragenden Gegenständen, z.B. Bekleidungsstücken, Schuhen, Schuheinlagen,
**gekennzeichnet durch**
eine Abbildung eines Druckverteilungsmusters, das **durch** mindestens eine mindestens zweidimensionale Abtastung einer Druckverteilung zwischen einem Körperteil der Person und einem im Wesentlichen festen Körper gewonnen ist, und in welcher verschieden hohe Drücke graphisch wiedergegeben sind.

2. Kennzeichnungseinrichtung nach Anspruch 1,
**dadurch gekennzeichnet, dass** die
verschieden hohe Drücke durch Flächenelemente verschiedener Farben und/oder verschiedener Schraffuren wiedergegeben sind.

3. Kennzeichnungseinrichtung nach einem der vorherigen Ansprüche,
**dadurch gekennzeichnet, dass**
die Abtastung der Druckverteilung eine Vielzahl von Abtastungen von verschiedenen Druckverteilungen umfasst, die während eines Bewegungsablaufes beim Aufbau und Abbau eines Kontaktes zwischen dem Körperteil und dem im Wesentlichen festen Körper auftreten.

4. Kennzeichnungseinrichtung nach Anspruch 3,
**dadurch gekennzeichnet, dass**
die Druckverteilungen Mittel- oder Spitzenwerte während des Bewegungsablaufes umfassen.

5. Kennzeichnungseinrichtung nach einem der vorherigen Ansprüche, zur Kennzeichnung eines Kleidungsstücks,
**dadurch gekennzeichnet, dass**
die Abbildung auf das Kleidungsstück durch Musteraufbringungsvorgänge, sowie Bedrucken oder durch Thermotransfer aufgedruckt ist.

6. Verwendung einer Kennzeichnungseinrichtung nach einem der Ansprüche 1 bis 4,
zur Kennzeichnung eines Kleidungsstücks, insbesondere eines Schuhs oder einer Einlegesohle, wobei das Druckverteilungsmuster unter einem Fuß beim Gehen gewonnen ist.

7. Verwendung einer Kennzeichnungseinrichtung nach einem der Ansprüche 1 bis 5,
zur Kennzeichnung eines Bekleidungsstücks, insbesondere einer Hose oder eines Handschuhs, wobei das Druckverteilungsmuster unter der Gesäßfläche beim Hinsetzen beziehungsweise an einer Handfläche beim Greifen gewonnen ist.

8. Verwendung nach einem der Ansprüche 6 oder 7,
wobei das Druckverteilungsmuster an der Person gewonnen ist, welche die Kennzeichnungseinrichtung verwendet.

9. Verfahren zur Herstellung einer Kennzeichnungseinrichtung zum individualisierenden Kennzeichnen von von einer Person am Körper zu tragenden Gegenständen, z.B. Bekleidungsstücken, Schuhen, Schuheinlagen, umfassend die Schritte
- Messen und Speichern eines Druckverteilungsmusters durch eine mindestens zweidimensionale Abtastung einer Druckverteilung zwischen einem Körperteil der Person und einem im Wesentlichen festen Körper
- Erstellen eines Druckes oder Abbildes der Druckverteilung, in welchem verschieden hohe Drücke graphisch wiedergegeben sind.

## Claims

1. Identification device for the individualised identification of objects worn by a person on the body, e.g. items of clothing, shoes, shoe inserts,
**characterised by**
an image of a pressure distribution pattern, which is obtained by at least one at least two-dimensional scanning of a pressure distribution between one part of the person's body and a substantially solid body, and in which varyingly high pressures are graphically reproduced.

2. Identification device according to Claim 1,
**characterised in that**
the varyingly high pressures are reproduced by surface elements of different colours and/or different hatchings.

3. Identification device according to one of the previous claims,
**characterised in that**
the scanning of the pressure distribution comprises a plurality of scannings of different pressure distributions, which occur during a movement sequence on the construction and reduction of a contact between the part of the body and the substantially solid body.

4. Identification device according to Claim 3,
**characterised in that**
the pressure distributions comprise mean or peak values during the movement sequence.

5. Identification device according to one of the previous claims, for identifying an item of clothing,
**characterised in that**
the image is printed onto the item of clothing by pattern application processes, as well as by printing or thermotransfer.

6. Use of an identification device according to one of the previous claims 1 to 4, for identifying an item of clothing, in particular a shoe or an insert sole, wherein the pressure distribution pattern is obtained below a foot when walking.

7. Use of an identification device according to one of the previous claims 1 to 5, for identifying a piece of clothing, in particular a pair of trousers or a glove, wherein the pressure distribution pattern is obtained below the seat area when sitting down or, respectively, on a hand surface when gripping.

8. Use according to one of the claims 6 or 7, wherein the pressure distribution pattern is obtained from the person who uses the identification device.

9. Method for manufacturing an identification device for the individualised identification of objects worn by a person on the body, e.g. items of clothing, shoes, shoe inserts, comprising the steps of
- measuring and storing a pressure distribution pattern by an at least two-dimensional scanning of a pressure distribution between one part of the person's body and a substantially solid body
- producing a print or image of the pressure distribution, in which varyingly high pressures are reproduced graphically.

## Revendications

1. Dispositif de marquage pour le marquage individualisant d'objets devant être portés au corps par une personne, par exemple des articles d'habillement, des chaussures, des semelles intérieures,
**caractérisé par** une représentation d'un motif de répartition de la pression, qui est obtenu par au moins un relevé au moins bidimensionnel d'une répartition de pression entre une partie du corps de la personne et un corps pour l'essentiel ferme, et dans lequel des pressions d'intensités différentes sont représentées graphiquement.

2. Dispositif de marquage selon la revendication 1, **caractérisé en ce que** les pressions d'intensités différentes sont reproduites par des éléments de surface de différentes couleurs et/ou présentant différentes hachures.

3. Dispositif de marquage selon l'une des revendications précédentes, **caractérisé en ce que** le relevé de la répartition de pression comprend une pluralité de relevés de différentes répartitions de pression qui apparaissent pendant une séquence de mouvement lors de l'instauration et de la suppression d'un contact entre la partie du corps et le corps pour l'essentiel ferme.

4. Dispositif de marquage selon la revendication 3, **caractérisé en ce que** les répartitions de pression comprennent des valeurs moyennes ou de crête pendant la séquence de mouvement.

5. Dispositif de marquage selon l'une des revendications précédentes, **caractérisé en ce que** la reproduction est imprimée sur l'article d'habillement par des opérations d'application de motif, ainsi que par impression ou par transfert thermique.

6. Utilisation d'un dispositif de marquage selon l'une des revendications 1 à 4 pour le marquage d'un article d'habillement, en particulier d'une chaussure ou d'une semelle intérieure, le motif de répartition de la pression étant obtenu sous un pied pendant la marche.

7. Utilisation d'un dispositif de marquage selon l'une des revendications 1 à 5 pour le marquage d'un article d'habillement, en particulier un pantalon ou un gant, le motif de répartition de la pression étant obtenu sous la surface d'assise quand on s'assoit, ou sur une surface de la main lors de la préhension.

8. Utilisation selon l'une des revendications 6 ou 7, dans laquelle le motif de répartition de la pression est obtenu sur la personne qui utilise le dispositif de marquage.

9. Procédé de fabrication d'un dispositif de marquage pour le marquage individualisant d'objets devant être portés au corps par une personne, par exemple des articles d'habillement, des chaussures, des semelles intérieures, comprenant les étapes de :
- mesure et enregistrement d'un motif de répartition de la pression par un relevé au moins bidimensionnel d'une répartition de pression entre une partie du corps de la personne et un corps pour l'essentiel ferme,
- réalisation d'une impression ou d'une représentation de la répartition de pression, dans laquelle des pressions d'intensités différentes sont représentées graphiquement.
